# EUROPEAN PATENT SPECIFICATION

(11) **EP 3 936 100 B1**
(45) Date of publication and mention of the grant of the patent: **10.09.2025**
(21) Application number: 20184374.5
(22) Date of filing: 07.07.2020
(51) Int. Cl.: A61F 13/42, A61F 13/84

(54) **MONITORING DEVICE BEING ATTACHABLE TO AN ABSORBENT ARTICLE AND COMMUNICATION METHOD THEREFOR**
ÜBERWACHUNGSVORRICHTUNG, DIE AN EINEM ABSORBIERENDEN ARTIKEL ANBRINGBAR IST, UND KOMMUNIKATIONSVERFAHREN DAFÜR
DISPOSITIF DE SURVEILLANCE POUVANT ÊTRE FIXÉ SUR UN ARTICLE ABSORBANT ET SON PROCÉDÉ DE COMMUNICATION

(43) Date of publication of application: 12.01.2022
(73) Proprietor: Ontex BV, 9255 Buggenhout (BE); Ontex Group NV, 9320 Erembodegem (BE)
(72) Inventor: HELLMOLD, Jens, 59269 Beckum (DE); HEIRMAN, Lisa, 9250 Waasmunster (BE); MARRES, Bert, 9300 Aalst (BE); HERMANS, Bert, 1860 Meise (BE); DE COSTER, Karel, 3050 Oud-Heverlee (BE)
(74) Representative: Saurat, Thibault

(56) References cited:
- EP-A1- 3 427 709
- WO-A1-2011/054045
- WO-A1-2015/094065
- WO-A1-2019/032991
- US-A1- 2005 156 744

## Description

The invention relates to a communication method for communication of a monitoring device being attachable to an absorbent article of a wearer with a back-end unit and a monitoring device being attachable to an absorbent article of a wearer.

Generally, in times of an increasing number of elderly and care-dependent people, there is a growing need of a communication method for communication of a monitoring device being attachable to an absorbent article of a wearer with a back-end unit and a monitoring device being attachable to an absorbent article of a wearer in order to handle incontinence in a particularly efficient manner.

WO 2014/177203 A1 relates to a method of monitoring an absorption state of an absorbent article, comprising providing a logger unit, acquiring the absorption state of the absorbent article and recording data indicating the acquired absorption state of the absorbent article in the logger unit. Acquiring the absorption state of the absorbent article and recording the data indicating the acquired absorption state of the absorbent article in the logger unit are continuously performed during a monitoring period of the absorbent article. Additionally, said document further relates to a logger unit for performing this method. Moreover, it relates to a method of processing data indicating an absorption state of an absorbent article, comprising receiving sets of data indicating the absorption state of the absorbent article. Each of the sets of data indicates the absorption state of the absorbent article over a portion of a continuous period of time. Disadvantageously, especially due to said continuity, a transmission of measurement results cannot be performed in an efficient and power-saving manner.

US2005156744 relates to a monitoring system for identifying a wet diaper by embedding an passive humidity sensor therein and attaching to the outside of the diaper a detachable transmitting module.

WO2011054045 relates to a system for monitoring incontinence in one or more subjects comprising display means.

WO2019032991 relates to a notification system that provides notification of patient events such as movement and/or incontinence.

EP3427709 relates to a wetness monitoring apparatus for incontinence surveillance.

WO2015094065 relates to a method for monitoring excrement data for seeking a pattern for a monitoring period, wherein said excrement data is associated with at least one absorbent article and a person wearing said at least one absorbent article.

Accordingly, there is an object to provide a communication method for communication of a monitoring device being attachable to an absorbent article of a wearer with a back-end unit and a monitoring device being attachable to an absorbent article of a wearer, whereby both a particularly high efficiency and a low power consumption are ensured.

This object is solved by the features of claim 1 for a communication method for communication of a monitoring device being attachable to an absorbent article of a wearer with a back-end unit and the features of claim 14 for a monitoring device being attachable to an absorbent article of a wearer. The dependent claims contain further developments.

According to a first aspect of the invention, a communication method for communication of a monitoring device being attachable to an absorbent article of a wearer with a back-end unit is provided. The communication method comprises the steps of measuring status information of the absorbent article and/or of the wearer after every first time interval in order to acquire corresponding measurement data for each respective time interval, wherein every first time interval (t1) is in the range of between 10 seconds and 10 minutes; and transmitting a trigger message comprising at least the latest measurement data from the monitoring device to the back-end unit if the latest measurement data is different from the corresponding previous measurement data of the respective previous time interval or a second time interval has been elapsed. It is noted that the second time interval may especially have been elapsed since the previous transmission of any message. The communication between the monitoring device and the back-end unit comprises or is based on at least one of wide area network, especially long range wide area network, Bluetooth, especially Bluetooth low energy, wireless local area network, Sigfox, LoRa, Internet-of-things, especially narrowband Internet-of-things, ZigBee, or any combination thereof. Advantageously, a particularly high efficiency and a low power consumption are ensured and complexity can be reduced.

According to a first preferred implementation form of the first aspect of the invention, the trigger message is an unconfirmed uplink message comprising solely data sent from the monitoring device to the back-end unit without an acknowledgement sent from the back-end unit to the monitoring device. In addition to this or as an alternative, the trigger message comprises solely non-critical information; wherein the non-critical information comprises at least one of voiding events, preferably detection of exudates, especially urine, bowel movements, temperature changes, or any combination thereof. Advantageously, for instance, efficiency of data transmission can further be increased.

According to a second preferred implementation form of the first aspect of the invention, the communication method further comprises the step of repeating the trigger message a certain times, preferably between 1 and 10 times, more preferably between 2 and 5 times, most preferably 3 times. Advantageously, for example, inefficiencies of data transmission can further be reduced.

According to a further preferred implementation form of the first aspect of the invention, in the case of at least two repetitions of the trigger message, between each repetition, there is a third time interval. Advantageously, for instance, power consumption can further be reduced.

According to a further preferred implementation form of the first aspect of the invention, the third time interval is lower than the first time interval. In addition to this or as an alternative, the third time interval is in the range of seconds, preferably between 1 and 10 seconds. Advantageously, for example, complexity can further be reduced, which leads to an increased efficiency.

According to a further preferred implementation form of the first aspect of the invention, the communication method further comprises the step of determining the certain times of repetitions of the trigger message and/or the third time interval on the basis of the risk for the trigger message not to arrive the back-end unit. Advantageously, for instance, flexibility can further be increased, thereby increasing efficiency.

According to a further preferred implementation form of the first aspect of the invention, the first time interval is in the range of seconds to minutes, preferably between 10 seconds and 10 minutes, more preferably between 30 seconds and 3 minutes, most preferably equal to 1 minute. Additionally or alternatively, the first time interval may especially depend on the respective measurement type. Advantageously, for example, power consumption can further be reduced.

According to a further preferred implementation form of the first aspect of the invention, the second time interval is larger than the first time interval, preferably is a multiple of the first time interval, more preferably is between 3 and 30 times the first time interval, most preferably is 15 times the first time interval. Additionally or alternatively, the second time interval may especially depend on the respective measurement type. Advantageously, for instance, efficiency can further be increased.

According to a further preferred implementation form of the first aspect of the invention, especially regardless of the first time interval and/or the second time interval, the communication method further comprises the step of transmitting an event message preferably comprising the latest measurement data from the monitoring device to the back-end unit. Advantageously, for example, inefficiencies of the data transmission can further be reduced.

According to a further preferred implementation form of the first aspect of the invention, the event message is a confirmed uplink message comprising data sent from the monitoring device to the back-end unit and a corresponding acknowledgement sent from the back-end unit to the monitoring device. In addition to this or as an alternative, the event message comprises critical information; wherein the critical information comprises at least one of disconnection and/or detachment of the monitoring device, detection of a fall of the wearer, no movement of the monitoring device within a predetermined time interval, irritation of the skin of the wearer, preferably a time being too long in one position, preferably a body posture and/or an orientation, with respect to the wearer and/or a time being too long since first exudated detection with respect to the wearer, low battery of the monitoring device, preferably a battery level of the monitoring device, preferably a battery level of the monitoring device of less than 10 per cent, or any combination thereof.Advantageously, for instance, efficiency of the data transmission can further be increased.

Further advantageously, with special respect to the above-mentioned no movement of the monitoring device within the predetermined time interval, it is noted that this can especially comprise or be an alarm. Said alarm may preferably be interpreted in a manner that the monitoring device is not worn and/or the absorbent article with the monitoring device is not worn and/or the wearer does not give vital signs anymore.

According to a further preferred implementation form of the first aspect of the invention, the communication method further comprises the step of retrying to transmit the event message a certain times, preferably between 1 and 20 times, more preferably between 3 and 10 times, most preferably 6 times, until the corresponding acknowledgement has been received by the monitoring device from the back-end unit. Advantageously, for example, not only efficiency but also power consumption can further be improved.

According to a further preferred implementation form of the first aspect of the invention, the communication method further comprises the step of determining the certain times of retrying to send the event message on the basis of the risk for the event message not to arrive the back-end unit. Advantageously, for instance, power consumption can further be reduced.

According to a further preferred implementation form of the first aspect of the invention, in the case that after the certain times of retrying to transmit the respective event message, no acknowledgment has been received by the monitoring device, the communication method further comprises the steps of storing the respective event message especially with the aid of a memory of the monitoring device, and/or transmitting the respective event message, preferably along with its corresponding timestamp, in combination with the next occurring message to be transmitted from the monitoring device to the back-end unit, especially regardless of which type the next occurring message is. Advantageously, for example, efficiency of data transmission can further be increased.

According to a second aspect of the invention, a monitoring device being attachable to an absorbent article of a wearer is provided. The monitoring device comprises a sensing unit and a communication unit connected to the sensing unit. In this context, the sensing unit is configured to measure status information of the absorbent article and/or of the wearer after every first time interval in order to acquire corresponding measurement data for each respective time interval. In addition to this, the communication unit is configured to transmit a trigger message comprising at least the latest measurement data to a back-end unit if the latest measurement data is different from the corresponding previous measurement data of the respective previous time interval or a second time interval has been elapsed. It is noted that the second time interval may especially have been elapsed since the previous transmission of any message. Advantageously, both a particularly high efficiency of the workflow and a low power consumption are ensured.

Further advantageously, it is noted that the monitoring device and/or the sensing unit may be configured to determine movements and/or postures, preferably body25 postures, and/or falls especially of the wearer. In this context, it might be particularly advantageous if the monitoring device and/or the sensing unit comprises an accelerometer and/or a gyroscope.30 Exemplary embodiments of the invention are now further explained with respect to the drawings by way of example only, and not for limitation. In the drawings:
- Fig. 1: shows a flow chart of an embodiment of the first aspect of the invention;
- Fig. 2: shows a flow chart comprising exemplary steps for monitoring a diaper with the aid of an inventive diaper monitoring device;
- Fig. 3: shows an exemplary flow chart based on Fig. 2 with detailed steps for the monitoring stage;
- Fig. 4: shows an exemplary timing diagram with respect to the inventive communication method;
- Fig. 5: shows an exemplary timing diagram comprising a close-up view with respect to a repetition of triggers;
- Fig. 6: shows an exemplary timing diagram comprising a close-up view with respect to a repetition of events;
- Fig. 7: shows an exemplary flow chart with respect to the transmission of measurement results;
- Fig. 8: shows an exemplary flow chart with respect to the transmission of the occurrence of events; and
- Fig. 9: shows an exemplary embodiment of the second aspect of the invention.

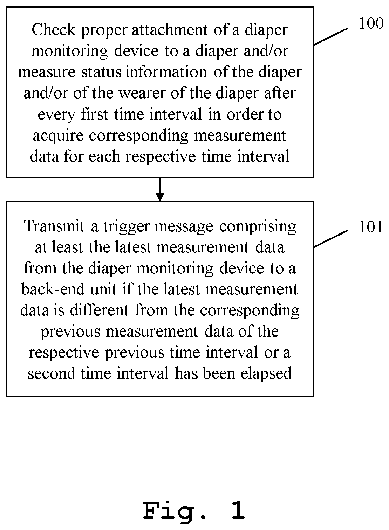

Firstly, Fig. 1 shows a flow chart of an embodiment of the inventive communication method for communication of a monitoring device, exemplarily a diaper monitoring device, being attachable to an absorbent article, exemplarily a diaper, of a wearer with a back-end unit.

According to a first step 100, the communication method comprises checking proper attachment of the diaper monitoring device to the diaper and/or measuring status information of the diaper and/or of the wearer after every first time interval (t1) in order to acquire corresponding measurement data for each respective time interval.

In addition to this, in accordance with a second step 101, the communication method comprises transmitting a trigger message comprising at least the latest measurement data from the diaper monitoring device to the back-end unit if the latest measurement data is different from the corresponding previous measurement data of the respective previous time interval or a second time interval (t2) has been elapsed.

It is noted that it might be particularly advantageous if the trigger message is an unconfirmed uplink message comprising solely data sent from the diaper monitoring device to the back-end unit without an acknowledgement sent from the back-end unit to the diaper monitoring device. In addition to this or as an alternative, the trigger message may comprise solely non-critical information especially with respect to the wearer and/or information not requiring an action and/or a feedback especially of the wearer and/or a caregiver of the wearer.

It is further noted that the communication method may further comprise the step of repeating the trigger message a certain times, preferably between 1 and 10 times, more preferably between 2 and 5 times, most preferably 3 times. Additionally or alternatively, the non-critical information may especially comprise at least one of voiding events, preferably presence and/or detection of exudates, especially urine and/or faeces, bowel movements, temperature changes, or any combination thereof.

Advantageously, especially in the case of at least two repetitions of the trigger message, between each repetition, there may be a third time interval t3. With respect to said third time interval t3, it is noted that the third time interval t3 may preferably be lower than the first time interval t1. In addition to this or as an alternative, the third time interval t3 may especially be in the range of seconds, preferably between 1 and 10 seconds.

Furthermore, the communication method may further comprise the step of determining the certain times of repetitions of the trigger message and/or the third time interval t3 on the basis of the risk for the trigger message not to arrive the back-end unit and/or an overall network load of a network comprising at least the diaper monitoring device and the back-end unit.

Moreover, with respect to the first time interval t1, it is noted that it might be particularly advantageous if the first time interval t1 is in the range of seconds to minutes, preferably between 10 seconds and 10 minutes, more preferably between 30 seconds and 3 minutes, most preferably equal to 1 minute. With respect to the second time interval t2, it is noted that the second time interval t2 may especially be larger than the first time interval t1, preferably is a multiple of the first time interval t1, more preferably is between 3 and 30 times the first time interval t1, most preferably is 15 times the first time interval t1.

In addition to this or as an alternative, especially regardless of the first time interval t1 and/or the second time interval t2, the communication method may further comprise the step of transmitting an event message preferably comprising the latest measurement data from the diaper monitoring device to the back-end unit. In this context, the event message may especially be a confirmed uplink message comprising data sent from the diaper monitoring device to the back-end unit and a corresponding acknowledgement sent from the back-end unit to the diaper monitoring device.

Additionally or alternatively, the event message may preferably comprise critical information especially with respect to the wearer and/or information requiring an action and/or a feedback especially of the wearer and/or a caregiver of the wearer. Moreover, the communication method may further comprise the step of retrying to transmit the event message certain times, preferably between 1 and 20 times, more preferably between 3 and 10 times, most preferably 6 times, until the corresponding acknowledgement has been received by the diaper monitoring device from the back-end unit.

In addition to this or as an alternative, the critical information may especially comprise at least one of disconnection and/or detachment of the diaper monitoring device, detection of a fall of the wearer, no movement of the monitoring device within a predetermined time interval, irritation of the skin of the wearer, preferably a time being too long in one position with respect to the wearer and/or a time being too long since first exudate detection with respect to the wearer, low battery of the diaper monitoring device, preferably a battery level of the diaper monitoring device of less than 10 per cent, or any combination thereof. In this context, it might be particularly advantageous if the communication method further comprises the step of determining the certain times of retrying to send the event message on the basis of the risk for the event message not to arrive the back-end unit and/or an overall network load of a network comprising at least the diaper monitoring device and the back-end unit.

Additionally or alternatively, especially in the case that after the certain times of retrying to transmit the respective event message, no acknowledgment has been received by the diaper monitoring device, the communication method may further comprise the steps of storing the respective event message especially with the aid of a memory of the diaper monitoring device, and/or transmitting the respective event message, preferably along with its corresponding timestamp, in combination with the next occurring message to be transmitted from the diaper monitoring device to the back-end unit, especially regardless of which type the next occurring message is.

In further addition to this or as a further alternative, the communication between the diaper monitoring device and the back-end unit may comprise or be based on at least one of wide area network, especially long range wide area network, Bluetooth, especially Bluetooth low energy, wireless local area network, LoRa, Sigfox, Internet-of-things, especially narrowband Internet-of-things, ZigBee, or any combination thereof.

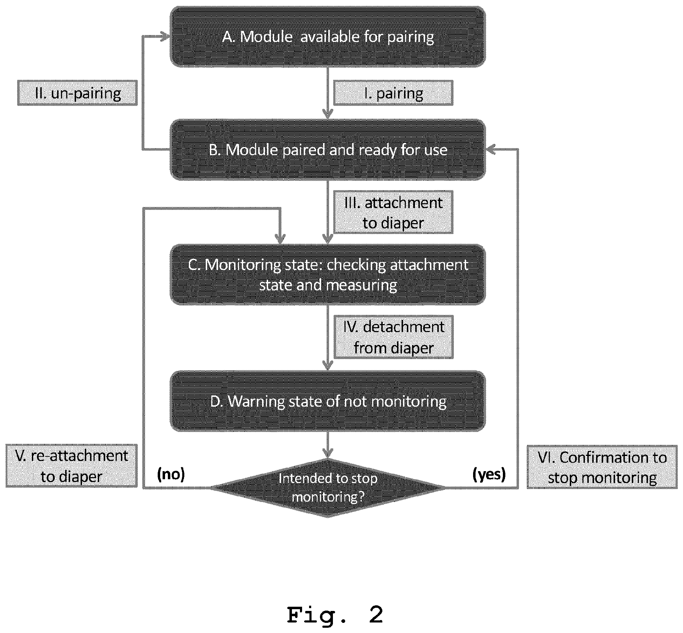

Now, with respect to Fig. 2, a flow chart comprising exemplary steps for monitoring a diaper with the aid of an inventive diaper monitoring device is shown. It should be mentioned that it might be particularly advantageous if the diaper monitoring devices are intended to be used in a care home setting. In this setting, multiple subjects would simultaneously make use of the system.

Besides, multiple subjects would interchangeably make use of diaper monitoring devices, meaning that any devices could be linked to any subject's personal profile through a process especially called "pairing" (I. pairing). When such a device is paired to the subject, it can be attached to a diaper worn by this subject (III. attachment to diaper), allowing to go into the monitoring state.

When the device is detached from the diaper (IV. detachment from diaper), it can either be used again through re-attachment (V. re-attachment to diaper), or the removal may have taken place intentionally so that it can be confirmed (VI. Confirmation to stop monitoring). Upon being back in its state ready for being used, it can either be used again or it can be un-paired (II. un-pairing) upon which the device becomes available for being paired again to the same or to another subject. Any of the steps (I) to (VI) will further on be named with "event" (cf. "event message") above.

With respect to the above-mentioned pairing to another subject, it is noted that a step of cleaning may advantageously be advised before using the respective device with another person.

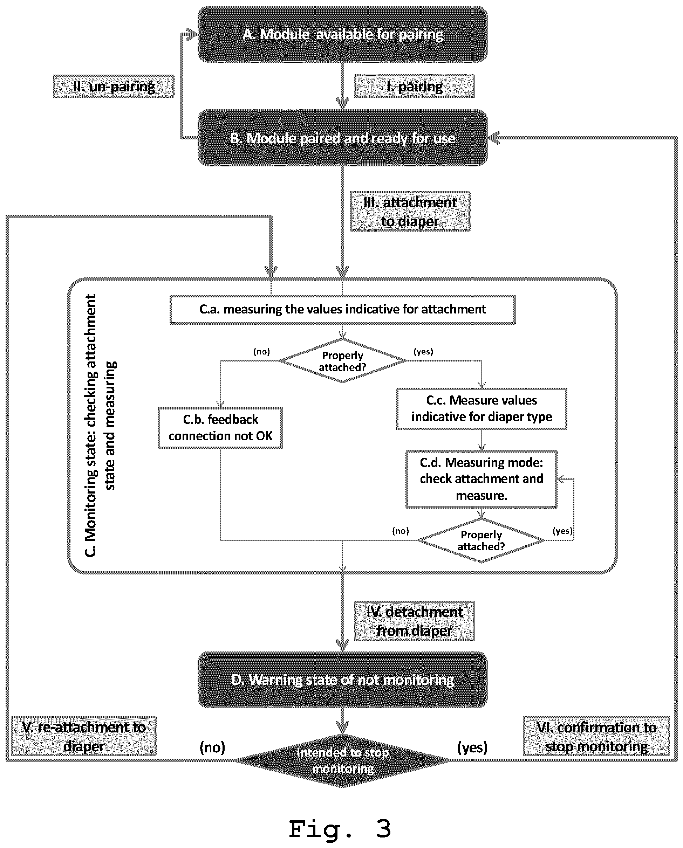

For what can happen during the monitoring state of a paired diaper monitoring device that is in use for the application of monitoring, especially wetness monitoring, within a diaper, an overview is given in detail within Fig. **3****.** It might be particularly advantageous if a product type identifier is included within the initial sensor status of a modified adult diaper, or within every following sensor status of a modified adult diaper along the use time, which can be measured by the diaper monitoring device after being attached to the diaper.

The logic of steps C.a, **C.b,** and C.c according to Fig. 3 can be handled within a diaper monitoring device and does especially not need interaction with a cloud application through wireless communication. Preferably, step C.d will repeatedly be executed, with a separation in between each execution of time interval t1 (cf. first interval as mentioned above).

Further on, each occurrence of this step will be covered within the term "measurement". Upon the occasion that a measurement is different versus a previous measurement, it would be the intention that this information gets transmitted, although for this example as well as for other examples the information can be considered to be non-critical. Such non-critical situations will now be covered within the term "trigger" (cf. trigger message as mentioned above).

Besides the examples for monitoring a diaper, especially wetness monitoring, as given in Fig. 2 and Fig. 3, other measurements can be performed by the diaper monitoring device. One example would be the indication of posture of a person, which can be considered as non-critical and can accordingly be captured within the term "measurement", the change of which would become a "trigger". Besides, other measurements may continuously be performed, such as the measurement of acceleration to detect the potential occurrence of falls. Upon the occurrence of a fall, the information would need to be transmitted directly by the diaper monitoring device, therefore such critical situations will accordingly be captured within the term "event".

In addition, specifically mentionable examples of measurements, triggers and events are given in the following:
With respect to measurements, a diaper monitoring device might read out the sensor status that corresponds to the wetness status once every minute. Specifically, this would be a series of resistive measurements. The series of measurement results could then be compared to the previous series of measurement results.

In addition to this or as an alternative, a diaper monitoring device might determine the orientation vs. the earth's gravity once for example every minute to conclude on the most likely posture of the wearer. This orientation could then be compared to the previously determined orientation. In this context, it might be particularly advantageous if an accelerometer and/or a gyroscope is/are employed.

Now, with respect to triggers, it is noted that each change in measurement result (when the new measurement result is different from the previous measurement result) could be a trigger. Additionally or alternatively, no change for a specific measurement type during a specified time interval (for instance, no change of posture during a certain time interval, exemplarily the past 5 hours) could be a trigger.

With respect to events, it is noted that a specific intended user interaction, such as the actions related to pairing of a diaper monitoring device to a person, could be an event. In addition to this or as an alternative, a specific unintended user interaction, such as the detachment of a diaper monitoring device from a diaper, could be an event. Further additionally or further alternatively, specific measurements, such as the detection of high acceleration indicative of a potential fall incident, could be events.

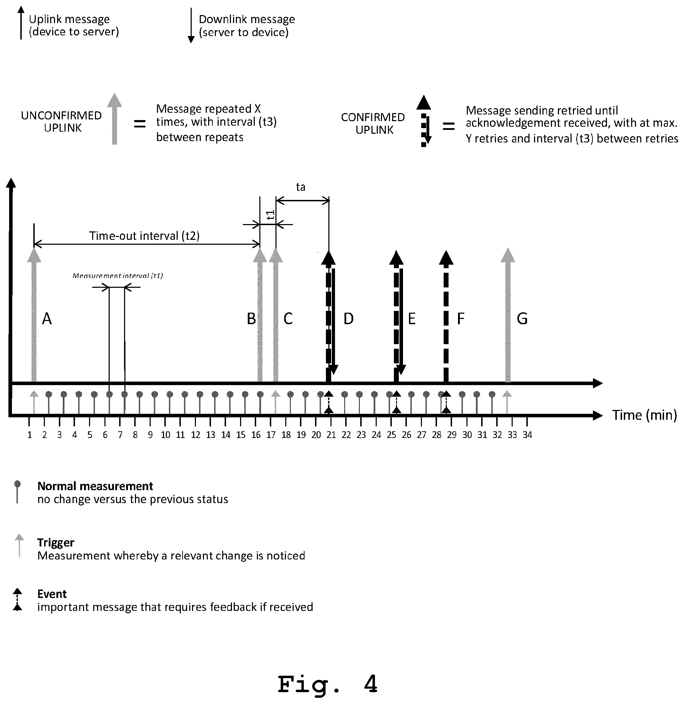

Now, with respect to Fig. **4****,** an exemplary timing diagram with respect to the inventive communication method is illustrated.

When arrived into the monitoring state, the diaper monitoring device performs measurements after every interval t1 (A). If the measurements are not showing any (relevant, the definition of relevant can be made individually for each measurement type) differences, the measurement will not result in any consequence. This is always the case, unless a time-out interval t2 has elapsed versus the previous transmission of any message or the previous measurement, in which case the information will anyway be forwarded and herewith treated the same way as a trigger (B).

After each measurement, the previous measurement information will especially be over-written within the diaper monitoring device. As such only the latest set of measurement data will preferably be stored within the diaper monitoring device. If the measurement shows a relevant difference versus the previous measurement, it will be treated as a trigger, regardless of the elapsed time in comparison to t2 (C).

Upon the occurrence of an event, which can happen at any time (D) regardless of the time ta elapsed versus the last data transmission and regardless of the time elapsed versus the last measurement, the message will be wirelessly transmitted along with the last set of measurement data. It should be noted that ta is a random value because an event can occur at any time, and specifically the related wireless transmission of said event will always take place (even when t2 has not elapsed yet).

Upon sending an update about the occurrence of an event or alert, an acknowledgement (downlink: back-end unit or server, respectively, to diaper monitoring device) will be requested from the server, to verify if the message has been received well (D). A new event can occur and will be transmitted immediately, regardless of whether the past uplink has been about a trigger (D) or the same or another event (E). When no acknowledgement has been received, the diaper monitoring device will send the event message again until an acknowledgement has been received, with a maximum of Y retries.

If after the Y'th retry still no acknowledgement has been received (F), the event will be stored in the diaper monitoring device's local memory and will be transmitted along with its timestamp in combination with the next occurring message, regardless of what type the next message is, such as the next message may be a trigger (G) with which the event message will then be combined. Preferably, for any trigger message as well as any attempt of an event message, the message will be sent repeatedly, with for triggers a number of repeats X and an interval between the repeated sending t3, and for events a number of repeats Y and an interval between the repeated sending t3.

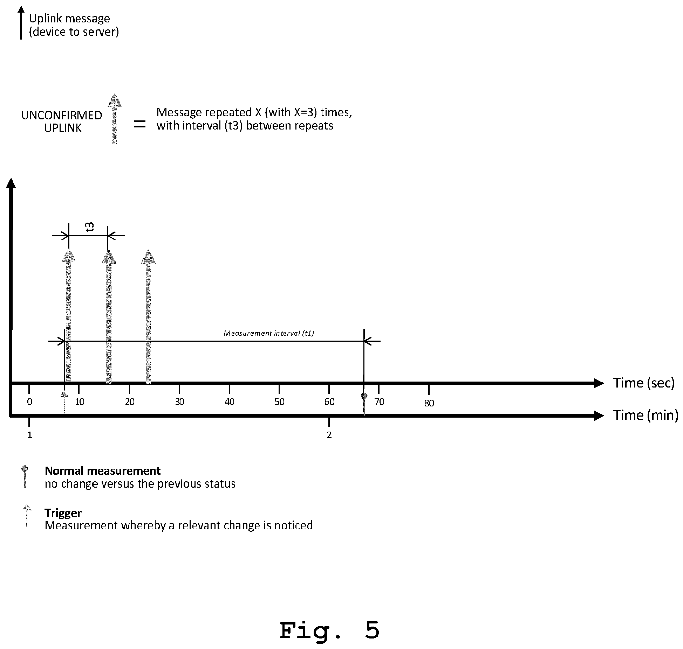

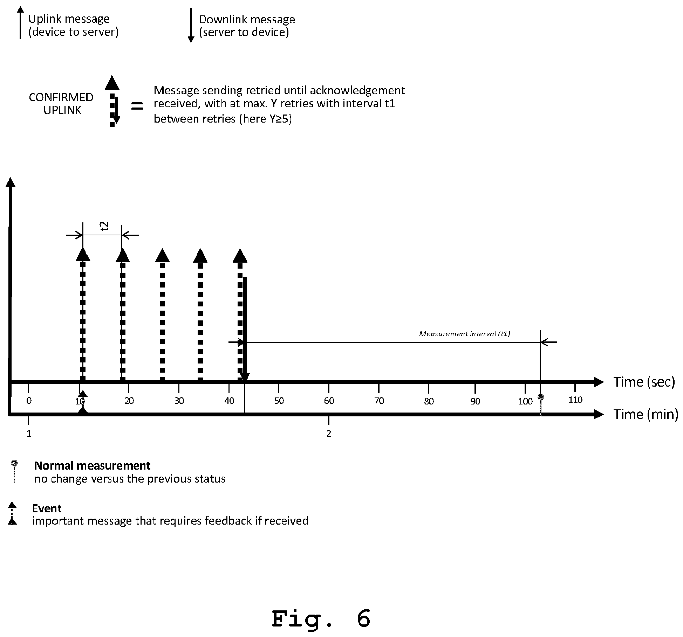

The number of repeated attempts X separated by t3 and the number of repeated attempts Y separated by t3 are illustrated by Fig. 5 and Fig. **6****.**

Advantageously, X and t3 are both determined by a trade-off between the risk for the message not to arrive at its destination versus the overall network load by all operational devices, especially diaper monitoring devices. X can take on any value, a particularly advantageous value would be ranging from 1 to 10, a safe value to optimize data traffic would be 2 to 5, most preferably 3. The interval t3 can take on any value and is typically in the range of seconds, typical values between 1 and 10 seconds.

As a preferable rule, t3 should be lower than t1. A preferred value for t1 depends on the type of measurements that are performed and is typically in the range of seconds to minutes, such as 10 seconds to 10 minutes, and 1 minute is a particularly preferred value. The second time parameter, t2, must be larger than t1 and is preferably a multiple of t2, such as a value between 3 and 30 times t1, and 15 times t1 is a particularly preferred value. The number Y of repeated attempts that can be made upon the occurrence of an event is, just like **X,** a trade-off between ensuring that the message arrives and the overall network load, and its value typically ranges from 1 to 20, preferably 3 to 10.

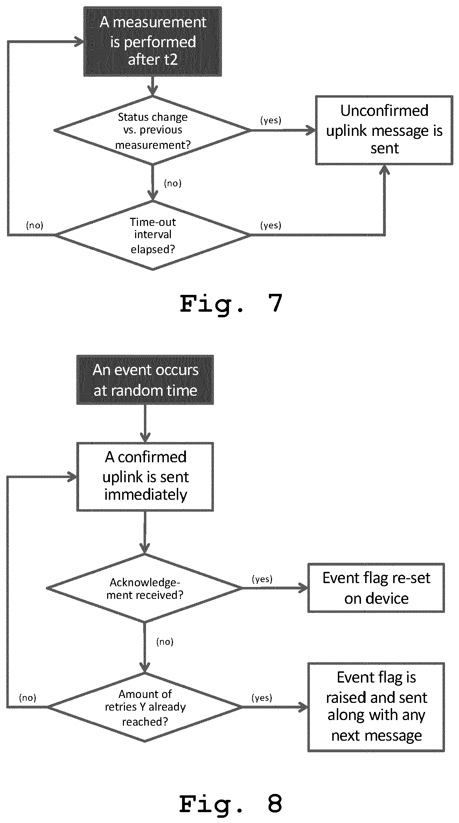

The logic for how and when to send an unconfirmed uplink according to Fig. 4 is further explained in Fig. **7****.** As a summary, no acknowledgement will be sent by the network server or back-end unit, respectively, about unconfirmed uplinks. So in worst case the message about a trigger does not arrive despite the X repeats. Only the previous status will be kept in the memory of the diaper monitoring device with the purpose to compare the actual status to the previous one. The server or back-end unit, respectively, will take on those messages, without determining the completeness of data throughout the monitoring period because to request this data back would unnecessarily consume data traffic for non-critical information.

The logic for how and when to send a confirmed uplink according to Fig. 4 is further explained in Fig. **8****.** Exemplarily, by raising a flag (especially within the diaper monitoring device or in a communication unit thereof) about the event in the unlikely case that no acknowledgement has been received after Y attempts, continuity about receiving all critical updates is ensured and the system, especially the diaper monitoring device, will verify automatically whether no information, preferably no critical information, is missing.

It is noted that per type of an event a flag can be raised so that even more than one unconfirmed event will be kept in a memory with its timestamp. Only a consecutive event of the same type will overwrite the first one.

For instance, if irritation of the skin of the wearer is detected, followed by a fall detection, both events will be kept in the memory (when receipt not confirmed by acknowledgement). If the person falls a second time, then the first fall will be overwritten by second incident.

It may further be noted that there can be cases where the network is unavailable, because of which unconfirmed uplinks are not received by the server or the back-end unit, respectively, and/or confirmed uplinks are not received by the system, especially the diaper monitoring device. Advantageously, by distinguishing between those that do not contain urgency and those cases that contain urgency, continuity of receiving all information that is critical (with or without a delay due to unavailability of the network) is ensured, without risking missing information due to network overloading.

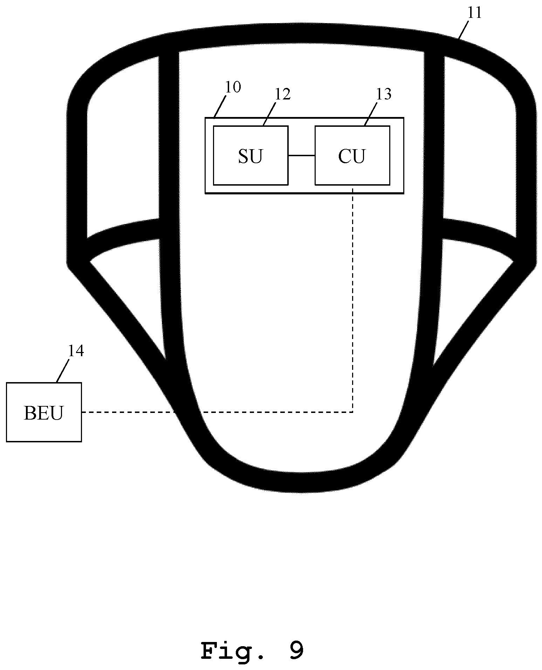

Finally, Fig. 9 illustrates an exemplary embodiment of an inventive monitoring device, exemplarily a diaper monitoring device 10, being attachable to an absorbent article, exemplarily a diaper 11, of a wearer. As it can be seen from Fig. 9, the diaper monitoring device 10 comprises a sensing unit 12, and a communication unit 13 connected to the sensing unit 12.

In this context, the sensing unit 12 is configured to check proper attachment of the diaper monitoring device 10 to the diaper 11 and/or to measure status information of the diaper 11 and/or of the wearer after every first time interval t1 in order to acquire corresponding measurement data for each respective time interval. In addition to this, the communication unit 13 is configured to transmit a trigger message comprising at least the latest measurement data to a back-end unit 14 if the latest measurement data is different from the corresponding previous measurement data of the respective previous time interval or a second time interval t2 has been elapsed.

With respect to the back-end unit 14, it is noted that there may be a connection device configured to connect the diaper monitoring device 10 to the back-end unit 14, such as a gateway if the back-end unit 14 is for example a cloud server. The gateway may then be configured to pass messages through from cloud to the device or from the device to the cloud, and it may independently send downlink messages to the device for confirmation of received uplinks.

It might be particularly advantageous if the trigger message is an unconfirmed uplink message comprising solely data sent from the diaper monitoring device 10 to the back-end unit 14 without an acknowledgement sent from the back-end unit 14 to the diaper monitoring device 10. In addition to this or as an alternative, the trigger message may comprise solely non-critical information especially with respect to the wearer and/or information not requiring an action and/or a feedback especially of the wearer and/or a caregiver of the wearer.

Furthermore, the diaper monitoring device 10, especially the communication unit 13 thereof, may be configured to repeat the trigger message a certain times, preferably between 1 and 10 times, more preferably between 2 and 5 times, most preferably 3 times. Additionally or alternatively, the non-critical information may comprise at least one of voiding events, preferably presence and/or detection of exudates, especially urine and/or faeces, bowel movements, temperature changes, or any combination thereof.

It is further noted that, especially in the case of at least two repetitions of the trigger message, between each repetition, there may be a third time interval t3. In this context, the third time interval t3 may be lower than the first time interval t1. Additionally or alternatively, the third time interval t3 may be in the range of seconds, preferably between 1 and 10 seconds.

Moreover, the diaper monitoring device 10, especially the communication unit 13 thereof, may be configured to determine the certain times of repetitions of the trigger message and/or the third time interval t3 on the basis of the risk for the trigger message not to arrive the back-end unit 14 and/or an overall network load of a network comprising at least the diaper monitoring device 10 and the back-end unit 14. It is noted that it might be particularly advantageous if the first time interval t1 is in the range of seconds to minutes, preferably between 10 seconds and 10 minutes, more preferably between 30 seconds and 3 minutes, most preferably equal to 1 minute.

In addition to this or as an alternative, the second time interval t2 may be larger than the first time interval t1, preferably is a multiple of the first time interval t1, more preferably is between 3 and 30 times the first time interval t1, most preferably is 15 times the first time interval t1. Furthermore, the diaper monitoring device 10, especially the communication unit 13 thereof, may be configured to, especially regardless of the first time interval t1 and/or the second time interval t2, transmit an event message comprising at least the latest measurement data from the diaper monitoring device 10 to the back-end unit 14.

In this context, the event message may especially be a confirmed uplink message comprising data sent from the diaper monitoring device 10 to the back-end unit 14 and a corresponding acknowledgement sent from the back-end unit 14 to the diaper monitoring device 10. In addition to this or as an alternative, the event message may comprise critical information especially with respect to the wearer and/or information requiring an action and/or a feedback especially of the wearer and/or a caregiver of the wearer.

Moreover, the diaper monitoring device 10, especially the communication unit 13 thereof, may be configured to retry to transmit the event message a certain times, preferably between 1 and 20 times, more preferably between 3 and 10 times, most preferably 6 times, until the corresponding acknowledgement has been received by the diaper monitoring device 10 (or by the communication unit 13, respectively) from the back-end unit 14.

Additionally or alternatively, the critical information may especially comprise at least one of disconnection and/or detachment of the diaper monitoring device 10, detection of a fall of the wearer, no movement of the monitoring device within a predetermined time interval, irritation of the skin of the wearer, preferably a time being too long in one position with respect to the wearer and/or a time being too long since first exudate detection with respect to the wearer, low battery of the diaper monitoring device 10, preferably a battery level of the diaper monitoring device 10 of less than 10 per cent, or any combination thereof.

Furthermore, the diaper monitoring device 10, especially the communication unit 13 thereof, may be configured to determine the certain times of retrying to send the event message on the basis of the risk for the event message not to arrive the back-end unit 14 and/or an overall network load of a network comprising at least the diaper monitoring device 10 and the back-end unit 14.

It is noted that especially in the case that after the certain times of retrying to transmit the respective event message, no acknowledgment has been received by the diaper monitoring device 10 (or by the communication unit 13, respectively), the diaper monitoring device 10, especially the communication unit 13 thereof, may be configured to store the respective event message especially with the aid of a memory of the diaper monitoring device 10, and/or to transmit the respective event message, preferably along with its corresponding timestamp, in combination with the next occurring message to be transmitted from the diaper monitoring device 10 to the back-end unit 14, especially regardless of which type the next occurring message is.

It is further noted that the communication between the diaper monitoring device 10 and the back-end unit 14 may comprise or be based on at least one of wide area network, especially long range wide area network, Bluetooth, especially Bluetooth low energy, wireless local area network, LoRa, Sigfox, Internet-of-things, especially narrowband Internet-of-things, ZigBee, or any combination thereof.

While various embodiments of the present invention have been described above, it should be understood that they have been presented by way of example only, and not limitation. Numerous changes to the disclosed embodiments can be made in accordance with the disclosure herein without departing from the scope of the invention. Thus, the breadth and scope of the present invention should not be limited by any of the above described embodiments. Rather, the scope of the invention should be defined in accordance with the following claims.

## Claims

1. A communication method for communication of a monitoring device (10) being attachable to an absorbent article of a wearer with a back-end unit (14), wherein the monitoring device (10) comprises a sensing unit (12), the communication method comprising the steps of:
the sensing unit measuring status information of the absorbent article and/or of the wearer after every first time interval (t1) in order to acquire corresponding measurement data for each respective time interval, wherein every first time interval (t1) is in the range of between 10 seconds and 10 minutes; and
transmitting a trigger message comprising at least the latest measurement data from the monitoring device (10) to the back-end unit (14) if the latest measurement data is different from the corresponding previous measurement data of the respective previous time interval or a second time interval (t2) has been elapsed; wherein the communication between the monitoring device (10) and the back-end unit (14) comprises or is based on at least one of wide area network, especially long range wide area network, Bluetooth, especially Bluetooth low energy, wireless local area network, Sigfox, LoRa, Internet-of-things, especially narrowband Internet-of-things, ZigBee, or any combination thereof.

2. The communication method according to claim 1,
wherein the trigger message is an unconfirmed uplink message comprising solely data sent from the monitoring device (10) to the back-end unit (14) without an acknowledgement sent from the back-end unit (14) to the monitoring device (10), and/or
wherein the trigger message comprises solely non-critical information; wherein the non-critical information comprises at least one of voiding events, preferably detection of exudates, especially urine, bowel movements, temperature changes, or any combination thereof.

3. The communication method according to claim 1 or 2,
wherein the communication method further comprises the step of repeating the trigger message a certain times, preferably between 1 and 10 times, more preferably between 2 and 5 times, most preferably 3 times.

4. The communication method according to claim 3,
wherein, in the case of at least two repetitions of the trigger message, between each repetition, there is a third time interval (t3).

5. The communication method according to claim 4,
wherein the third time interval (t3) is lower than the first time interval (t1), and/or
wherein the third time interval (t3) is in the range of seconds, preferably between 1 and 10 seconds.

6. The communication method according to any of the claims 3 to 5,
wherein the communication method further comprises the step of determining the certain times of repetitions of the trigger message and/or the third time interval (t3) on the basis of the risk for the trigger message not to arrive the back-end unit (14).

7. The communication method according to any of the claims 1 to 6,
wherein the first time interval (t1) is in the range of seconds to minutes, preferably between 10 seconds and 10 minutes, more preferably between 30 seconds and 3 minutes, most preferably equal to 1 minute.

8. The communication method according to any of the claims 1 to 7,
wherein the second time interval (t2) is larger than the first time interval (t1), preferably is a multiple of the first time interval (t1), more preferably is between 3 and 30 times the first time interval (t1), most preferably is 15 times the first time interval (t1).

9. The communication method according to any of the claims 1 to 8,
wherein, regardless of the first time interval (t1) and/or the second time interval (t2), the communication method further comprises the step of transmitting an event message preferably comprising the latest measurement data from the monitoring device (10) to the back-end unit (14).

10. The communication method according to claim 9,
wherein the event message is a confirmed uplink message comprising data sent from the monitoring device (10) to the back-end unit (14) and a corresponding acknowledgement sent from the back-end unit (14) to the monitoring device (10), and/or
wherein the event message comprises critical information;wherein the critical information comprises at least one of disconnection and/or detachment of the monitoring device (10), detection of a fall of the wearer, no movement of the monitoring device within a predetermined time interval, irritation of the skin of the wearer, preferably a time being too long in one position, preferably a body posture and/or an orientation, with respect to the wearer and/or a time being too long since first exudated detection with respect to the wearer, low battery of the monitoring device (10), preferably a battery level of the monitoring device (10) of less than 10 per cent, or any combination thereof.

11. The communication method according to claim 9 or 10,
wherein the communication method further comprises the step of retrying to transmit the event message a certain times, preferably between 1 and 20 times, more preferably between 3 and 10 times, most preferably 6 times, until the corresponding acknowledgement has been received by the monitoring device (10) from the back-end unit (14).

12. The communication method according to claim 11,
wherein the communication method further comprises the step of determining the certain times of retrying to send the event message on the basis of the risk for the event message not to arrive the back-end unit (14).

13. The communication method according to claim 11 or 12,
wherein in the case that after the certain times of retrying to transmit the respective event message no acknowledgment has been received by the monitoring device (10), the communication method further comprises the steps of:
storing the respective event message especially with the aid of a memory of the monitoring device (10), and/or
transmitting the respective event message, preferably along with its corresponding timestamp, in combination with the next occurring message to be transmitted from the monitoring device (10) to the back-end unit (14), regardless of which type the next occurring message is.

14. A monitoring device (10) being attachable to a absorbent article of a wearer, the monitoring device (10) comprising:
a sensing unit (12), and
a communication unit (13) connected to the sensing unit (12),
wherein the sensing unit (12) is configured to measure status information of the absorbent article and/or of the wearer after every first time interval (t1) in order to acquire corresponding measurement data for each respective time interval, and
wherein the communication unit (13) is configured to transmit a trigger message comprising at least the latest measurement data to a back-end unit (14) if the latest measurement data is different from the corresponding previous measurement data of the respective previous time interval or a second time interval (t2) has been elapsed.

## Patentansprüche

1. Kommunikationsverfahren zur Kommunikation einer Überwachungsvorrichtung (10), die an einem saugfähigen Artikel eines Trägers befestigbar ist, mit einer hinteren Endeinheit (14), wobei die Überwachungsvorrichtung (10) eine Sensoreinheit (12) umfasst, wobei das Kommunikationsverfahren die folgenden Schritte umfasst:
die Sensoreinheit misst Statusinformationen des saugfähigen Artikels und/oder des Trägers nach jedem ersten Zeitintervall (t1), um entsprechende Messdaten für jedes jeweilige Zeitintervall zu erfassen, wobei jedes erste Zeitintervall (t1) im Bereich zwischen 10 Sekunden und 10 Minuten liegt; und
Übertragung einer Auslösungsnachricht, die zumindest die neuesten Messdaten enthält, von der Überwachungsvorrichtung (10) an die hintere Endeinheit (14), wenn sich die neuesten Messdaten von den entsprechenden vorherigen Messdaten des jeweils vorherigen Zeitintervalls unterscheiden oder ein zweites Zeitintervall (t2) abgelaufen ist;
wobei die Kommunikation zwischen der Überwachungsvorrichtung (10) und der hinteren Endeinheit (14) mindestens eine der folgenden Technologien umfasst oder darauf basiert: Weitverkehrsnetz, insbesondere Long Range Wide Area Network, Bluetooth, insbesondere Bluetooth Low Energy, drahtloses lokales Netzwerk, Sigfox, LoRa, Internet der Dinge, insbesondere Narrowband Internet of Things, ZigBee oder jede Kombination davon.

2. Kommunikationsverfahren nach Anspruch 1,
wobei die Auslösungsnachricht eine unbestätigte Uplink-Nachricht ist, die ausschließlich Daten umfasst, die von der Überwachungsvorrichtung (10) an die hintere Endeinheit (14) gesendet werden, ohne dass eine Empfangsbestätigung von der hinteren Endeinheit (14) an die Überwachungsvorrichtung (10) gesendet wird, und/oder
wobei die Auslösungsnachricht ausschließlich nichtkritische Informationen umfasst; wobei die nichtkritischen Informationen mindestens eine der folgenden Angaben enthalten: Entleerungsereignisse, vorzugsweise Detektion von Exsudaten, insbesondere Urin, Stuhlgang, Temperaturänderungen oder jede Kombination davon.

3. Kommunikationsverfahren nach Anspruch 1 oder **2,**
wobei das Kommunikationsverfahren ferner den Schritt umfasst, die Auslösungsnachricht eine bestimmte Anzahl von Malen zu wiederholen, vorzugsweise zwischen 1 und 10 Mal, noch bevorzugter zwischen 2 und 5 Mal, und am bevorzugtesten 3 Mal.

4. Kommunikationsverfahren nach Anspruch **3,**
wobei im Fall von mindestens zwei Wiederholungen der Auslösungsnachricht zwischen den einzelnen Wiederholungen jeweils ein drittes Zeitintervall (t3) liegt.

5. Kommunikationsverfahren nach Anspruch 4,
wobei das dritte Zeitintervall (t3) kürzer als das erste Zeitintervall (t1) ist, und/oder
wobei das dritte Zeitintervall (t3) im Sekundenbereich liegt, vorzugsweise zwischen 1 und 10 Sekunden.

6. Kommunikationsverfahren nach einem der Ansprüche 3 bis 5, wobei das Kommunikationsverfahren ferner den Schritt umfasst, die Anzahl der Wiederholungen der Auslösungsnachricht und/oder das dritte Zeitintervall (t3) basierend auf dem Risiko zu bestimmen, dass die Auslösungsnachricht die hintere Endeinheit (14) nicht erreicht.

7. Kommunikationsverfahren nach einem der Ansprüche 1 bis 6, wobei das erste Zeitintervall (t1) im Bereich von Sekunden bis Minuten liegt, vorzugsweise zwischen 10 Sekunden und 10 Minuten, noch bevorzugter zwischen 30 Sekunden und 3 Minuten, und am bevorzugtesten gleich 1 Minute ist.

8. Kommunikationsverfahren nach einem der Ansprüche 1 bis 7, wobei das zweite Zeitintervall (t2) größer als das erste Zeitintervall (t1) ist, vorzugsweise ein Vielfaches des ersten Zeitintervalls (t1), noch bevorzugter zwischen dem 3- bis 30-fachen des ersten Zeitintervalls (t1), und am bevorzugtesten das 15-fache des ersten Zeitintervalls (t1).

9. Kommunikationsverfahren nach einem der Ansprüche 1 bis 8, wobei - unabhängig vom ersten Zeitintervall (t1) und/oder dem zweiten Zeitintervall (t2) - das Kommunikationsverfahren ferner den Schritt umfasst, eine Ereignisnachricht zu übertragen, die vorzugsweise die neuesten Messdaten der Überwachungsvorrichtung (10) an die hintere Endeinheit (14) enthält.

10. Kommunikationsverfahren nach Anspruch **9,** wobei die Ereignisnachricht eine bestätigte Uplink-Nachricht ist, die Daten enthält, die von der Überwachungsvorrichtung (10) an die hintere Endeinheit (14) gesendet werden, sowie eine entsprechende Empfangsbestätigung, die von der hinteren Endeinheit (14) an die Überwachungsvorrichtung (10) gesendet wird, und/oder wobei die Ereignisnachricht kritische Informationen enthält; wobei die kritischen Informationen mindestens eine der folgenden Angaben enthalten: Trennung und/oder Ablösung der Überwachungsvorrichtung (10), Sturzerkennung des Trägers, keine Bewegung der Überwachungsvorrichtung innerhalb eines vorgegebenen Zeitintervalls, Hautreizung des Trägers, vorzugsweise eine zu lange Verweildauer in einer bestimmten Position, vorzugsweise Körperhaltung und/oder Orientierung in Bezug auf den Träger, und/oder ein zu langer Zeitraum seit der ersten Detektion von Exsudaten in Bezug auf den Träger, niedriger Batteriestand der Überwachungsvorrichtung (10), vorzugsweise ein Batteriestand von weniger als 10 **%,** oder jede Kombination davon.

11. Kommunikationsverfahren nach Anspruch 9 oder 10, wobei das Kommunikationsverfahren ferner den Schritt umfasst, zu versuchen, die Ereignisnachricht eine bestimmte Anzahl von Malen erneut zu übertragen, vorzugsweise zwischen 1 und 20 Mal, noch bevorzugter zwischen 3 und 10 Mal, und am bevorzugtesten 6 Mal, bis die entsprechende Empfangsbestätigung von der hinteren Endeinheit (14) bei der Überwachungsvorrichtung (10) eingegangen ist.

12. Kommunikationsverfahren nach Anspruch 11, wobei das Kommunikationsverfahren ferner den Schritt umfasst, die Anzahl der Wiederholungen für die Übertragung der Ereignisnachricht auf der Grundlage des Risikos zu bestimmen, dass die Ereignisnachricht die hintere Endeinheit (14) nicht erreicht.

13. Kommunikationsverfahren nach Anspruch 11 oder 12, wobei, wenn nach der bestimmten Anzahl von Wiederholungen der Übertragung der jeweiligen Ereignisnachricht keine Empfangsbestätigung von der Überwachungsvorrichtung (10) empfangen wurde, das Kommunikationsverfahren ferner die folgenden Schritte umfasst:
speichern der jeweiligen Ereignisnachricht, insbesondere mit Hilfe eines Speichers der Überwachungsvorrichtung (10), und/oder
übertragen der jeweiligen Ereignisnachricht, vorzugsweise zusammen mit dem zugehörigen Zeitstempel, in Kombination mit der nächsten zu übertragenden Nachricht von der Überwachungsvorrichtung (10) an die hintere Endeinheit (14), unabhängig vom Typ der nächsten Nachricht.

14. Überwachungsvorrichtung (10), die an einem saugfähigen Artikel eines Trägers befestigbar ist, wobei die Überwachungsvorrichtung (10) umfasst:
eine Sensoreinheit (12), und
eine Kommunikationseinheit (13), die mit der Sensoreinheit (12) verbunden ist,
wobei die Sensoreinheit (12) dafür eingerichtet ist, nach jedem ersten Zeitintervall (t1) Statusinformationen des saugfähigen Artikels und/oder des Trägers zu messen, um entsprechende Messdaten für jedes jeweilige Zeitintervall zu erfassen, und
wobei die Kommunikationseinheit (13) dafür eingerichtet ist, eine Auslösungsnachricht, die zumindest die neuesten Messdaten enthält, an eine hintere Endeinheit (14) zu übertragen, wenn sich die neuesten Messdaten von den entsprechenden vorherigen Messdaten des jeweiligen vorherigen Zeitintervalls unterscheiden oder ein zweites Zeitintervall (t2) abgelaufen ist.

## Revendications

1. Procédé de communication pour la communication d'un dispositif de surveillance (10) pouvant être fixé à un article absorbant d'un porteur avec une unité d'extrémité arrière (14), ledit dispositif de surveillance (10) comprenant une unité de détection (12), le procédé de communication comprenant les étapes suivantes :
l'unité de détection mesure des informations d'état de l'article absorbant et/ou du porteur après chaque premier intervalle de temps (t1), afin d'acquérir des données de mesure correspondantes pour chaque intervalle de temps respectif, chaque premier intervalle de temps (t1) étant compris entre 10 secondes et 10 minutes ; et
transmission d'un message de déclenchement comprenant au moins les dernières données de mesure du dispositif de surveillance (10) à l'unité d'extrémité arrière (14), si les dernières données de mesure sont différentes des données de mesure précédentes correspondantes de l'intervalle de temps précédent respectif ou si un second intervalle de temps (t2) s'est écoulé ;
la communication entre le dispositif de surveillance (10) et l'unité d'extrémité arrière (14) comprenant ou étant basée sur au moins un des éléments suivants : réseau étendu, notamment réseau étendu à longue portée, Bluetooth, notamment Bluetooth basse énergie, réseau local sans fil, Sigfox, LoRa, Internet des objets, notamment Internet des objets à bande étroite, ZigBee, ou toute combinaison de ceux-ci.

2. Procédé de communication selon la revendication 1,
dans lequel le message de déclenchement est un message de liaison montante non confirmé comprenant uniquement des données envoyées du dispositif de surveillance (10) à l'unité d'extrémité arrière (14), sans accusé de réception envoyé par l'unité d'extrémité arrière (14) au dispositif de surveillance (10), et/ou
dans lequel le message de déclenchement comprend uniquement des informations non critiques ; lesdites informations non critiques comprenant au moins un des éléments suivants : événements d'évacuation, de préférence détection d'exsudats, notamment urine, selles, variations de température, ou toute combinaison de ceux-ci.

3. Procédé de communication selon la revendication 1 ou **2,**
dans lequel le procédé de communication comprend en outre l'étape de répétition du message de déclenchement un certain nombre de fois, de préférence entre 1 et 10 fois, plus préférablement entre 2 et 5 fois, et très préférablement 3 fois.

4. Procédé de communication selon la revendication **3,**
dans lequel, en cas d'au moins deux répétitions du message de déclenchement, un troisième intervalle de temps (t3) est prévu entre chaque répétition.

5. Procédé de communication selon la revendication **4,**
dans lequel le troisième intervalle de temps (t3) est inférieur au premier intervalle de temps (t1), et/ou dans lequel le troisième intervalle de temps (t3) est de l'ordre de quelques secondes, de préférence compris entre 1 et 10 secondes.

6. Procédé de communication selon l'une quelconque des revendications 3 à 5,
dans lequel le procédé de communication comprend en outre l'étape de détermination du nombre de répétitions du message de déclenchement et/ou du troisième intervalle de temps (t3) sur la base du risque que le message de déclenchement n'atteigne pas l'unité d'extrémité arrière (14).

7. Procédé de communication selon l'une quelconque des revendications 1 à 6,
dans lequel le premier intervalle de temps (t1) est de l'ordre de quelques secondes à quelques minutes, de préférence compris entre 10 secondes et 10 minutes, plus préférablement entre 30 secondes et 3 minutes, et très préférablement égal à 1 minute.

8. Procédé de communication selon l'une quelconque des revendications 1 à 7,
dans lequel le second intervalle de temps (t2) est supérieur au premier intervalle de temps (t1), de préférence est un multiple du premier intervalle de temps (t1), plus préférablement compris entre 3 et 30 fois le premier intervalle de temps (t1), et très préférablement égal à 15 fois le premier intervalle de temps (t1).

9. Procédé de communication selon l'une quelconque des revendications 1 à 8,
dans lequel, indépendamment du premier intervalle de temps (t1) et/ou du second intervalle de temps (t2), le procédé de communication comprend en outre l'étape de transmission d'un message d'événement comprenant de préférence les dernières données de mesure du dispositif de surveillance (10) à l'unité d'extrémité arrière (14).

10. Procédé de communication selon la revendication 9,
dans lequel le message d'événement est un message de liaison montante confirmé comprenant des données envoyées du dispositif de surveillance (10) à l'unité d'extrémité arrière (14) et un accusé de réception correspondant envoyé de l'unité d'extrémité arrière (14) au dispositif de surveillance (10), et/ou
dans lequel le message d'événement comprend des informations critiques ; lesdites informations critiques comprenant au moins l'un des éléments suivants : déconnexion et/ou détachement du dispositif de surveillance (10), détection d'une chute du porteur, absence de mouvement du dispositif de surveillance pendant un intervalle de temps prédéterminé, irritation de la peau du porteur, de préférence un temps passé trop long dans une même position, de préférence une posture et/ou orientation corporelle, par rapport au porteur, et/ou un temps trop long écoulé depuis la première détection d'exsudats par rapport au porteur, batterie faible du dispositif de surveillance (10), de préférence un niveau de batterie du dispositif de surveillance (10) inférieur à 10 %, ou toute combinaison de ceux-ci.

11. Procédé de communication selon la revendication 9 ou 10,
dans lequel le procédé de communication comprend en outre l'étape de réessayer de transmettre le message d'événement un certain nombre de fois, de préférence entre 1 et 20 fois, plus préférablement entre 3 et 10 fois, et très préférablement 6 fois, jusqu'à ce que l'accusé de réception correspondant soit reçu par le dispositif de surveillance (10) depuis l'unité d'extrémité arrière (14).

12. Procédé de communication selon la revendication 11, dans lequel le procédé de communication comprend en outre l'étape de détermination du nombre de tentatives de retransmission du message d'événement sur la base du risque que le message d'événement n'atteigne pas l'unité d'extrémité arrière (14).

13. Procédé de communication selon la revendication 11 ou 12,
dans lequel, si après le nombre défini de tentatives de retransmission du message d'événement respectif aucun accusé de réception **n'a** été reçu par le dispositif de surveillance (10), le procédé de communication comprend en outre les étapes suivantes :
stocker le message d'événement respectif, notamment à **l'aide** d'une mémoire du dispositif de surveillance (10), et/ou
transmettre le message d'événement respectif, de préférence accompagné de son horodatage correspondant, en combinaison avec le prochain message à transmettre du dispositif de surveillance (10) à l'unité d'extrémité arrière (14), quel que soit le type du prochain message.

14. Dispositif de surveillance (10) pouvant être fixé à un article absorbant porté par un porteur, le dispositif de surveillance (10) comprenant :
une unité de détection (12), et
une unité de communication (13) connectée à l'unité de détection (12),
dans lequel l'unité de détection (12) est conçue pour mesurer des informations d'état de l'article absorbant et/ou du porteur après chaque premier intervalle de temps (t1) afin d'acquérir des données de mesure correspondantes pour chaque intervalle de temps respectif, et dans lequel l'unité de communication (13) est conçue pour transmettre un message de déclenchement comprenant au moins les dernières données de mesure à une unité **d'extrémité** arrière (14) si les dernières données de mesure sont différentes des données de mesure précédentes correspondantes de l'intervalle de temps précédent respectif ou si un second intervalle de temps (t2) **s'est** écoulé.
